# EUROPEAN PATENT APPLICATION

(11) **EP 4 799 622 A1**
(43) Date of publication of application: **02.09.2026**
(21) Application number: 24881542.5
(22) Date of filing: 21.10.2024
(51) Int. Cl.: A61K 31/495, A61K 47/02, A61K 47/00, A61K 9/00, A61K 9/20, A61K 9/16, A61P 25/24

(54) **VORTIOXETINE PHARMACEUTICAL COMPOSITION, PREPARATION METHOD THEREFOR, AND USE THEREOF**

(30) Priority: 24.10.2023 CN 202311382475
(71) Applicant: Seasons Biotechnology (Taizhou) Co., Ltd., Taizhou, Zhejiang 318099 (CN)
(72) Inventor: JIA, Qiang, Taizhou, Zhejiang 318099 (CN); MA, Tianhua, Taizhou, Zhejiang 318099 (CN); LAI, Guangjian, Taizhou, Zhejiang 318099 (CN); WANG, Ru, Taizhou, Zhejiang 318099 (CN); CHEN, Qinchun, Taizhou, Zhejiang 318099 (CN); WANG, Yunzhong, Taizhou, Zhejiang 318099 (CN)
(74) Representative: dompatent
(86) International application number: PCT/CN2024/126024
(87) International publication number: WO 2025/087177

(57) **Abstract**

Disclosed is a vortioxetine nitrosamine impurity SJ003-139, which is present in both the vortioxetine active pharmaceutical ingredient and pharmaceutical compositions. The content of the impurity increases during the preparation and storage of the vortioxetine active pharmaceutical ingredient and pharmaceutical compositions. Therefore, a pharmaceutical composition with a low content of nitrosamine impurities, a preparation method therefore and use thereof are disclosed in the present disclosure. The pharmaceutical composition of the present disclosure has the advantage that the content of nitrosamine impurities is low, ensuring the safety of medication in patients.

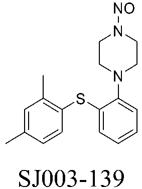

## Description

### FIELD OF THE INVENTION

The present disclosure relates to the technical field of pharmaceutical preparations, in particular to a vortioxetine pharmaceutical composition, especially to a vortioxetine pharmaceutical composition with low nitrosamine impurity content, as well as a preparation method and application thereof.

### BACKGROUND OF THE INVENTION

Vortioxetine has the chemical name 1-[2-(2,4-dimethylphenylsulfanyl)phenyl]piperazine. As a multi-target antidepressant, it exerts both 5-HT₃ receptor antagonism and 5-HT₁A receptor agonism, and is used for the treatment of major depressive disorder. At present, vortioxetine hydrobromide is used clinically as the active pharmaceutical ingredient. It was co-developed by Takeda Pharmaceutical Company Limited and H. Lundbeck A/S, and approved by the U.S. Food and Drug Administration (FDA) on September 30, 2013, under the trade name Brintellix. Four approved strengths are available: 5 mg, 10 mg, 15 mg, and 20 mg, in the dosage form of film coated tablets. The structural formula of vortioxetine is shown below:

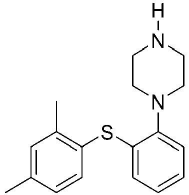

Nitrosamine compounds, (R₁)(R₂)N-N=O are genotoxic substances, and some have been classified as possible or potential human carcinogens by the International Agency for Research on Cancer (IARC). ICH guidelines recommend that any known mutagenic or carcinogenic substances, including nitrosamine compounds, be controlled at intake levels associated with negligible human cancer risk.

As reported in the literature "Reactive Impurities in Excipients: Profiling, Identification and Mitigation of Drug-Excipient Incompatibility (Wu, et al., AAPS PharmSciTech, 2011, 12(4), 1248-1263)", some commonly used pharmaceutical excipients contain -NO₂ groups, which can react with secondary amines to form nitrosamine impurities. Vortioxetine contains a piperazine ring in its structure, and the secondary amine moiety may generate nitrosamine impurities during the preparation of the compound, as well as during the preparation and storage of pharmaceutical formulations.

To date, no literature has been reported concerning the control of nitrosamine impurity levels in vortioxetine pharmaceutical compositions.

In view of the toxicity of nitrosamine impurities, there is an urgent need for a vortioxetine pharmaceutical composition in which the nitrosamine impurity content can be controlled at a low level.

### SUMMARY OF THE INVENTION

During the research on vortioxetine pharmaceutical compositions, the present applicant discovered that a nitrosamine impurity is produced during the preparation process of vortioxetine drug substance. Furthermore, the content of the nitrosamine impurity increases during the preparation and storage of vortioxetine pharmaceutical compositions, which presents a potential risk to patient medication safety. Accordingly, the present disclosure provides the structure of the nitrosamine impurity and a method for determining the content of the impurity in vortioxetine or a vortioxetine pharmaceutical composition. The present disclosure further provides a pharmaceutical composition having a low nitrosamine impurity content and high safety, as well as a preparation method and use thereof.

One or more embodiments of the present disclosure provide pharmaceutical composition , which comprises vortioxetine or a pharmaceutically acceptable salt thereof and an alkaline excipient, wherein the content of nitrosamine impurity in the pharmaceutical composition is no more than 20 ppm, and the structural formula of nitrosamine impurity is shown as Formula SJ003-139:

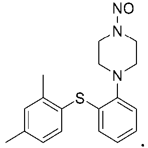

In some embodiments of the present disclosure, wherein alkaline excipient is at least one selected from trisodium phosphate, disodium hydrogen phosphate, tripotassium phosphate, dipotassium hydrogen phosphate, calcium phosphate, calcium carbonate, potassium carbonate, sodium carbonate, sodium bicarbonate, potassium citrate, sodium acetate, calcium hydroxide, sodium hydroxide, potassium hydroxide, and meglumine, preferably sodium carbonate.

In some embodiments of the present disclosure, pharmaceutical composition further comprises an antioxidant having a phenol structure: butylated hydroxytoluene or butylated hydroxyanisole, preferably butylated hydroxytoluene.

In some embodiments of the present disclosure, the nitrosamine impurity content of pharmaceutical composition is no more than 19 ppm.

In some embodiments of the present disclosure, the nitrosamine impurity content of pharmaceutical composition is no more than 18 ppm.

In some embodiments of the present disclosure, the nitrosamine impurity content of pharmaceutical composition is no more than 17 ppm.

In some embodiments of the present disclosure, the nitrosamine impurity content of pharmaceutical composition is no more than 16 ppm.

In some embodiments of the present disclosure, the nitrosamine impurity content of pharmaceutical composition is no more than 15 ppm.

In some embodiments of the present disclosure, the nitrosamine impurity content of pharmaceutical composition is no more than 14 ppm.

In some embodiments of the present disclosure, the nitrosamine impurity content of pharmaceutical composition is no more than 13 ppm.

In some embodiments of the present disclosure, the nitrosamine impurity content of pharmaceutical composition is no more than 12 ppm.

In some embodiments of the present disclosure, the nitrosamine impurity content of pharmaceutical composition is no more than 11 ppm.

In some embodiments of the present disclosure, the nitrosamine impurity content of pharmaceutical composition is no more than 10 ppm.

In some embodiments of the present disclosure, the nitrosamine impurity content of pharmaceutical composition is no more than 9 ppm.

In some embodiments of the present disclosure, the nitrosamine impurity content of pharmaceutical composition is no more than 8 ppm.

In some embodiments of the present disclosure, the nitrosamine impurity content of pharmaceutical composition is no more than 7 ppm.

In some embodiments of the present disclosure, the nitrosamine impurity content of pharmaceutical composition is no more than 6.7 ppm.

In some embodiments of the present disclosure, the nitrosamine impurity content of pharmaceutical composition is no more than 6 ppm.

In some embodiments of the present disclosure, the nitrosamine impurity content of pharmaceutical composition is no more than 5 ppm.

In some embodiments of the present disclosure, the nitrosamine impurity content of pharmaceutical composition is no more than 4 ppm.

In some embodiments of the present disclosure, the nitrosamine impurity content of pharmaceutical composition is no more than 3 ppm.

In some embodiments of the present disclosure, the nitrosamine impurity content of pharmaceutical composition is no more than 2 ppm.

In some embodiments of the present disclosure, wherein pharmaceutical composition further comprises two or more pharmaceutically acceptable excipients selected from the group consisting of: a filler, a disintegrant, glidant, and a lubricant;
wherein diluent is one or two selected from mannitol, sorbitol, xylitol, polyethylene glycol, hydroxypropyl cellulose, hypromellose, microcrystalline cellulose, and anhydrous lactose, preferably one or two of mannitol or microcrystalline cellulose, more preferably mannitol;
wherein disintegrant is at least two selected from crospovidone, croscarmellose sodium, and sodium carboxymethyl starch, preferably crospovidone and croscarmellose sodium;
wherein glidant is at least two selected from talc, silicon dioxide, and magnesium silicate, preferably talc and silicon dioxide;
wherein lubricant is at least one selected from magnesium stearate, stearic acid, sodium lauryl sulfate, and talc, preferably magnesium stearate;

Optionally, the pharmaceutical composition further comprises at least one of a flavoring agent and a taste masking agent;
wherein the flavoring agent is at least one selected from L-menthol, neotame, monosodium glutamate, and saccharin, preferably L-menthol or neotame;
wherein the taste masking agent is at least one selected from amino methacrylate copolymer, polyacrylic resin II, polyacrylic resin III, and polyvinyl acetate phthalate, preferably amino methacrylate copolymer.

In some embodiments of the present disclosure, pharmaceutical composition comprises:
vortioxetine or a pharmaceutically acceptable salt thereof 10~20%;
alkaline excipient 0.2~5%;
antioxidant 0.02~0.1%;
filler 30~85%;
disintegrant 1.5~15%;
glidant 1.0~12%;
lubricant 0.25~5%;
taste-masking agent 1~25%.

In some embodiments of the present disclosure, wherein pharmaceutical composition comprises:
vortioxetine or a pharmaceutically acceptable salt thereof 10~20%;
anhydrous sodium carbonate 0.2~5%;
butylated hydroxytoluene 0.02~0.1%;
mannitol 30~85%;
crospovidone 1~5%;
croscarmellose sodium 0.5~8%;
talc 1~10%;
silicon dioxide 0.1~1.0%;
magnesium stearate 0.25~5%;
amino methacrylate copolymer 1~25%;
wherein the pharmaceutically acceptable salt is hydrochloride, hydrobromide, or
hemihydrobromide.

In some embodiments of the present disclosure, wherein the pharmaceutical composition is an oral solid preparation, preferably a tablet or granule.

In some embodiments of the present disclosure, wherein the pharmaceutical composition is an orally disintegrating tablet.

In some embodiments of the present disclosure, provide method for treating depression disorder or cognitive impairment in a subject, comprising administering to the subject the pharmaceutical composition.

### Advantages of the present disclosure:

The present applicant discovered that water is inevitably used during the preparation of vortioxetine, and water contains nitrite. Therefore, vortioxetine can react with nitrite in water to produce nitrosamine impurity SJ003-139. Furthermore, during the preparation of vortioxetine pharmaceutical compositions, some pharmaceutical excipients (such as anhydrous lactose, microcrystalline cellulose, and hydroxypropyl cellulose) also contain -NO₂ groups. During the preparation and storage of vortioxetine pharmaceutical compositions comprising such excipients, vortioxetine reacts with -NO₂ groups to produce nitrosamine impurity SJ003-139.

The present applicant found that the addition of an alkaline excipient (such as anhydrous sodium carbonate) or an antioxidant to the vortioxetine pharmaceutical composition can inhibit the conversion of the active ingredient vortioxetine into nitrosamine impurity SJ003-139. The combined addition of an alkaline excipient and an antioxidant exhibits an even better effect in inhibiting the conversion of the active ingredient vortioxetine into nitrosamine impurity SJ003-139. For patients, this reduces genotoxic impurities in the drug and helps ensure medication safety.

To improve taste or enhance medication compliance, one or more flavoring agents selected from the group consisting of L-menthol, neotame, monosodium glutamate, and saccharin may be optionally added to the tablets of the present invention.

### EXAMPLES

The following describes the embodiments of the invention in detail with reference to specific examples, but those skilled in the art should understand that the examples are only used to illustrate the invention, and should not be regarded as limiting the scope of the invention._{∘}

Examples of fillers in the present disclosure include mannitol 100SD, purchased from Roquette Freres; and microcrystalline cellulose, model PH102 or 112, purchased from Microcellulose Weissenborn GmbH + Co. KG.

Examples of disintegrants in the present disclosure include crospovidone XL-10, purchased from Chongqing Starck Riedema Material Technology Co., Ltd.

Examples of glidants in the present disclosure include silicon dioxide 244FP, purchased from Grace GmbH.

Examples of lubricants in the present disclosure include magnesium stearate, purchased from Beijing Fengli Company.

In the present disclosure, "BHT" is the abbreviation for butylated hydroxytoluene.

When detecting the SJ003-139 impurity in the present invention, in addition to determining the initial impurity content of the tablets, the SJ003-139 impurity content is determined after the number of days of storage testing in the commercial packaging or proposed commercial packaging.
ppm: parts per million, one part per million; 1 ppm is equivalent to one part in a million.

Unless otherwise specified, the reagents or instruments used are all commercially available products obtained through regular channels.

### Example 1: Preparation of Nitrosamine Impurity SJ003-139

To a reaction flask were added 2 g of vortioxetine and 10 ml of ethanol, followed by 1.7 g of concentrated hydrochloric acid under stirring, and the mixture was cooled to below 30 °C. 10 ml of NaNO₂ solution (0.5 g/ml) was added dropwise. After completion of the reaction as monitored by TLC, the mixture was cooled to precipitate a solid, which was filtered and dried in vacuo to obtain 1.5 g of the target compound.

¹H NMR (600 MHz, Chloroform-d) δ7.38 (d, J = 7.8 Hz, 1H), 7.17 (s, 1H), 7.12-7.07 (m, 1H), 7.05 (d, J = 7.7 Hz, 1H), 7.03-6.99 (m, 1H), 6.96-6.89 (m, 1H), 6.57 (dd, J = 7.9, 1.3 Hz, 1H), 4.48-4.39 (m, 2H), 4.03 (s, 2H), 3.32-3.23 (m, 2H), 3.04 (t, J = 5.2 Hz, 2H), 2.37 (s, 3H), 2.34 (s, 3H).

### Example 2: Preparation of Vortioxetine Hemihydrobromide

To a reaction flask were added 10 g of vortioxetine and 100 ml of ethyl acetate. Under stirring, 2.82 g of hydrobromic acid (48% wt) was added. The mixture was subjected to suction filtration and dried in vacuo to obtain 10.80 g of vortioxetine hemihydrobromide.

### LC-MS Analysis Conditions:

Instrument: Shimadzu LC-20ADXR LC with Sciex API 4000 QTrap or equivalent;
Column: Agilent Poroshell 120 EC C18, 3.0 mm × 100 mm, 2.7 µm;

### Chromatographic Parameters:

Flow rate: 0.5 mL/min;
Column temperature: 35 °C;
Sample chamber temperature: 25 °C;
Injection volume: 5 µL;
Diluent: Acetonitrile: Water = 60:40 (V/V);
Mobile phase A: Ammonium acetate buffer;
Mobile phase B: Acetonitrile.

### Gradient Elution Program:

| Time (min) | Mobile Phase A (%) | Mobile Phase B (%) |
|---|---|---|
| 0 | 60 | 40 |
| 5 | 15 | 85 |
| 10 | 15 | 85 |
| 11 | 60 | 40 |
| 15 | 60 | 40 |

### Mass Spectrometry Operating Parameters:

| | Parameter |
|---|---|
| Polarity | Positive |
| Nebulizer Gas (psi) | 45 |
| Heating Gas (psi) | 50 |
| Curtain Gas (psi) | 30 |
| Ionization Temperature (°C) | 450 |
| Spray Voltage (V) | 5000 |

### MRM Channels Parameters: Multiple Reaction Monitoring

| compound | Precursor Ion (m/z) | Product Ion (m/z) | Dwell (ms) | DP (V) | EP (V) | CE (V) | CXP (V) |
|---|---|---|---|---|---|---|---|
| SJ003-139 | 328.3 | 298.2 | 100 | 60 | 10 | 16 | 15 |

A sample of the vortioxetine hemihydrobromide prepared in this example was prepared at a concentration of 2 mg/mL.

Upon detection under the above-described LC-MS analysis conditions, the content of SJ003-139 in the vortioxetine hemihydrobromide prepared in this example was 0.23 ppm.

### Example 3: Preparation of Vortioxetine Hydrobromide

To a reaction flask were added 10.0 g of 1-[2-(2,4-dimethylphenylsulfanyl)phenyl]piperazine and 100 mL of ethyl acetate. The mixture was heated to 60 °C, and 5.65 g of hydrobromic acid aqueous solution (48 wt.%) was added. A large amount of solid gradually precipitated. The mixture was cooled to 0-10 °C, filtered with suction, and dried in vacuo to obtain 12.30 g of vortioxetine hydrobromide as a white solid.

The content of SJ003-139 in the vortioxetine hydrobromide was determined to be 0.25 ppm by LC-MS under the same conditions as those described in Example 2.

### Example 4: Preparation of Vortioxetine Hydrochloride

To a reaction flask were added 10.0 g of 1-[2-(2,4-dimethylphenylsulfanyl)phenyl]piperazine and 80 mL of ethyl acetate. The mixture was heated to 60 °C, and 3.40 g of concentrated hydrochloric acid was added dropwise. After completion of the addition, a large amount of solid gradually precipitated. The mixture was cooled to 0-10 °C, filtered with suction, and dried in vacuo to obtain 10.30 g of vortioxetine hydrochloride as a white solid.

The content of SJ003-139 in the vortioxetine hydrochloride was determined to be 0.28 ppm by LC-MS under the same conditions as those described in Example 2.

### Example 5

### Preparation Method of Tablets:

1.Preparation of inner granules: The taste masking agent and the antioxidant are dissolved in anhydrous ethanol. The active pharmaceutical ingredient and glidant are added under stirring, followed by fluidized bed granulation, drying, and sieving, to obtain the inner granules.
2.Preparation of tablets: The filler, disintegrant, and alkaline excipient are mixed to obtain Mixture I. Mixture I, the lubricant, and the inner granules obtained in Step 1 are mixed to obtain Mixture II. Mixture II is sieved and sized, then compressed into tablets to obtain the desired tablets.

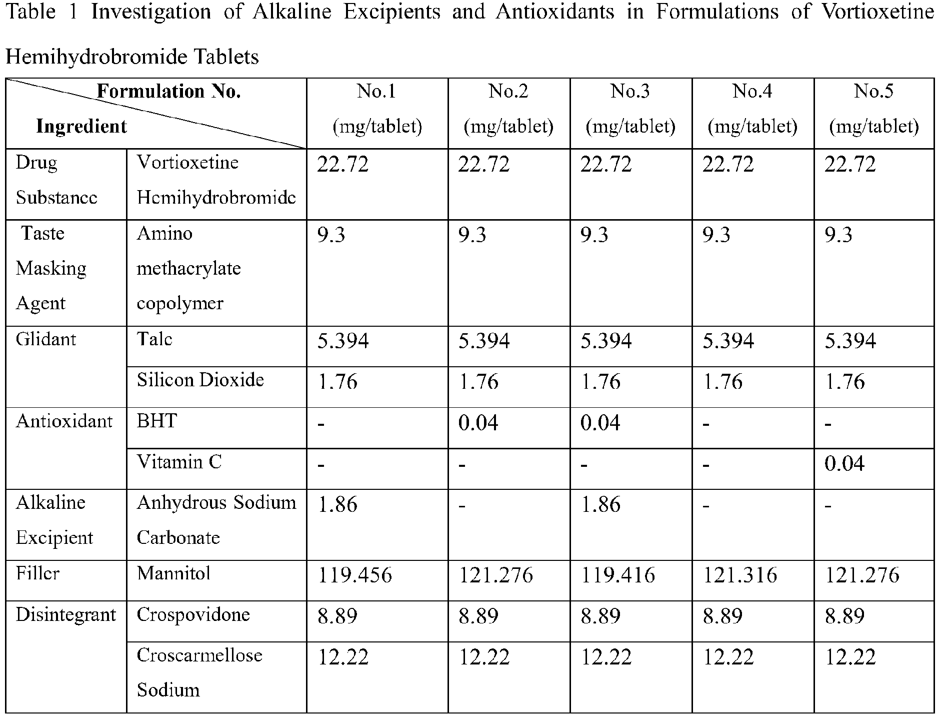

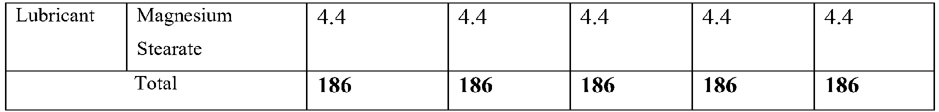

Table 1 provides formulations of vortioxetine hemihydrobromide tablets containing anhydrous sodium carbonate, BHT, vitamin C, anhydrous sodium carbonate and BHT, and neither anhydrous sodium carbonate nor BHT, respectively.

In Table 1, the weight of vortioxetine hemihydrobromide is equivalent to 20 mg of vortioxetine per tablet.

The initial tablets and the tablets stored at 60 °C for 10 days of each formulation in Table 1 were tested, and the content of impurity SJ003-139 is shown in Table 2,

**Table 2 Impurity Content of Tablets No.1-No.5**

| Formulation No. | Antioxidant and Alkaline Excipient Content(wt%) | SJ003-139 Impurity Content (ppm) | 60 °C, 10 days (ppm) |
|---|---|---|---|
| 1 | 1% Anhydrous Sodium Carbonate | 0.36 | 2.5 |
| 2 | 0.02%BHT | 0.435 | 7.1 |
| 3 | 0.02%BHT+1%Anhydrous Sodium Carbonate | 0.273 | 1.6 |
| 4 | - | 2.5 | 11.57 |
| 5 | 0.02%Vitamin C | 2.37 | 8.1 |

The symbol "-" in the table indicates the absence of antioxidant and alkaline excipient.

As can be seen from Table 2, Formulation No.3 containing both BHT and anhydrous sodium carbonate exhibited the lowest SJ003-139 impurity content, at 0.273 ppm.

A comparison of the impurity levels in Formulations No.2 and No.4 shows that the addition of BHT as an antioxidant effectively inhibits or reduces impurity formation. The impurity content after storage at 60 °C for 10 days is higher in the formulation without BHT than in the formulation containing BHT. A comparison of Formulations No.2 and No.5 shows that the use of BHT as an antioxidant resulted in a significantly lower impurity content than the use of vitamin C as an antioxidant, indicating that BHT is more effective than vitamin C in inhibiting impurity formation in the tablets.

A comparison of Formulations No.1 and No.4 shows that the addition of only 1% anhydrous sodium carbonate results in a significantly lower impurity content than the formulation without antioxidant or anhydrous sodium carbonate.

Five batches of Formulation No.3 tablets were packaged in proposed commercial packaging and stored long-term for 32 months at 25 °C/40% RH. The SJ003-139 content was determined to be 1.5-1.9 ppm.

Unopened reference listed drug (RLD) Brintellix^{®} tablets (manufacture date: December 2020) were stored for 32 months, and the SJ003-139 content was determined to be 9.21 ppm.

The reference listed drug Brintellix^{®} tablets do not contain anhydrous sodium carbonate or BHT, which further demonstrates that the addition of an antioxidant and an alkaline excipient inhibits the formation of SJ003-139.

The results in Table 2 demonstrate that anhydrous sodium carbonate as an alkaline excipient and BHT as an antioxidant each inhibit impurity formation in the tablets. The combined use of anhydrous sodium carbonate and BHT in the tablets provides a superior effect in inhibiting nitrosamine impurity formation compared to the use of either component alone.

### Example 6

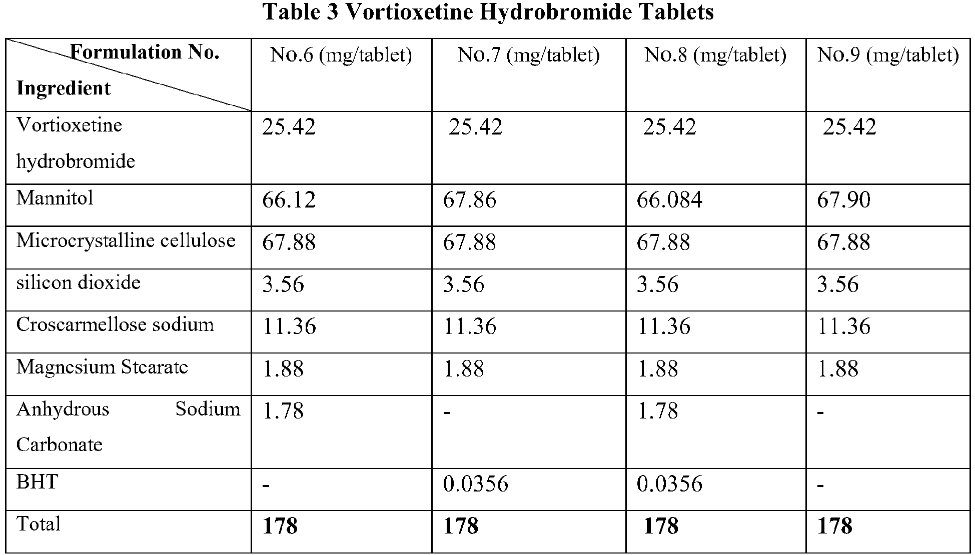

Table 3 provides the components of vortioxetine hydrobromide tablets containing anhydrous sodium carbonate, BHT, anhydrous sodium carbonate and BHT, and neither anhydrous sodium carbonate nor BHT, respectively.

In Table 3, the weight of vortioxetine hydrobromide is equivalent to 20 mg of vortioxetine per tablet. The tablets were prepared according to the preparation method of vortioxetine hemihydrobromide tablets described in Example 5.

**Table 4 Initial Impurity Content of Tablets No.6-No.9**

| **Formulation No.** | Antioxidant and Alkaline Excipient Content(wt%) | SJ003-139 Impurity Content (ppm) |
|---|---|---|
| 6 | 1%Anhydrous Sodium Carbonate | 0.988 |
| 7 | 0.02%BHT | 1.267 |
| 8 | 0.02%BHT+1%Anhydrous Sodium Carbonate | 0.644 |
| 9 | - | 25.711 |

Tablets No.6~No. 9 were stored at 60 °C for 10 days.

The SJ003-139 impurity content of Tablets No.6 ~No. 8 was no more than 6.7 ppm, while the SJ003-139 impurity content of Tablet No. 9 was 32.577 ppm after storage at 60 °C for 10 days. The SJ003-139 impurity contents of the four tablets in Table 4 demonstrate that anhydrous sodium carbonate as an alkaline excipient and BHT as an antioxidant can each effectively inhibit the formation of impurity SJ003-139 in vortioxetine hydrobromide tablets.

### Example 7

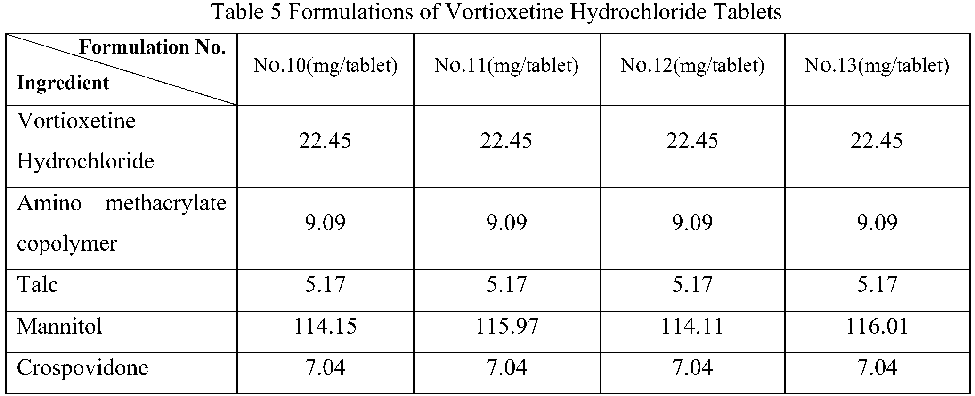

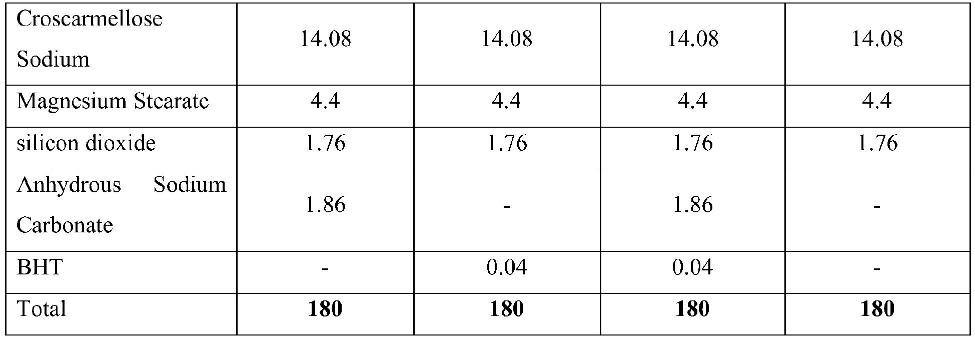

Table 5 provides the formulations of vortioxetine hydrochloride tablets containing anhydrous sodium carbonate, BHT, anhydrous sodium carbonate and BHT, and neither anhydrous sodium carbonate nor BHT, respectively; the weight of vortioxetine hydrochloride in Table 5 is 20 mg per tablet calculated as vortioxetine, and the preparation of the tablets refers to the preparation method for vortioxetine hemihydrobromide tablets in Example 5.

The initial contents of impurity SJ003-139 in Tablets No. 10 to No.13 are 1.210 ppm, 1.531 ppm, 0.933 ppm, and 25.711 ppm, respectively. After storage at 60°C for 10 days, the content of impurity SJ003-139 in Tablets No.10 to No.12 does not exceed 6.7 ppm, and the content of impurity SJ003-139 in Tablet No.13 is 34.731 ppm. In Example 6, the active ingredient is vortioxetine hydrobromide, and the impurity content is relatively higher than that of vortioxetine hemihydrobromide tablets in Example 5; the active ingredient in this Example is vortioxetine hydrochloride, and the content of impurity SJ003-139 is higher than that of vortioxetine hydrobromide and vortioxetine hemihydrobromide tablets.

### Example 8

Table 6 presents the formulations in which the active pharmaceutical ingredient is vortioxetine hemihydrobromide and the alkaline excipient is calcium phosphate.

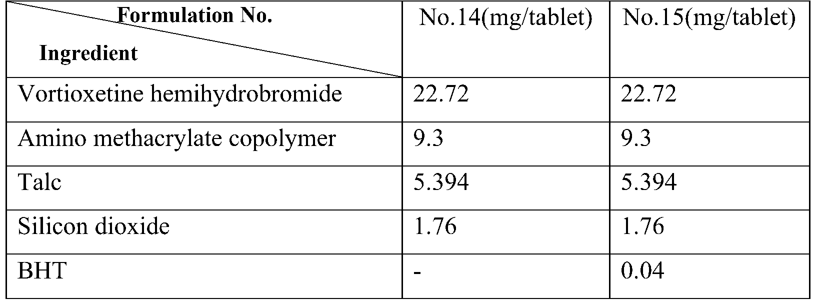

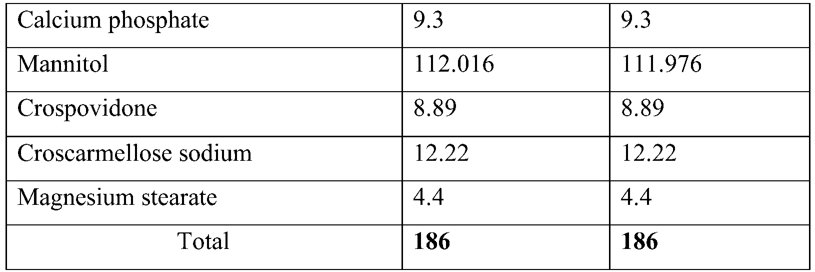

Table 6 shows that the initial contents of impurity SJ003-139 in the tablets formulated as Formulations No.14 and No.15 are 0.198 ppm and 0.11 ppm, respectively, while the contents of impurity SJ003-139 after storage at 60°C for 10 days are 3.1 ppm and 2.7 ppm, respectively.

### Example 9

Table 7 presents the formulations wherein the active pharmaceutical ingredient is vortioxetine hemihydrobromide and the alkaline excipient is sodium acetate.

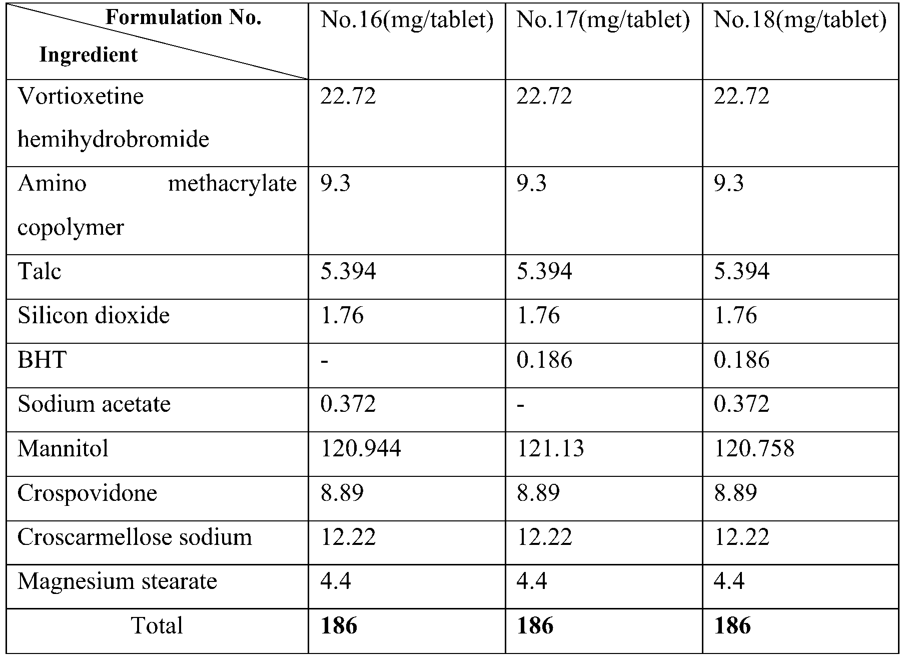

Table 7 shows that the initial contents of impurity SJ003-139 in the tablets formulated as Formulations No.16, No.17, and No.18 are 0.683 ppm, 0.664 ppm, and 0.439 ppm, respectively. After storage at 60°C for 10 days, the content of impurity SJ003-139 in each is less than 6.7 ppm. Formulation No.2 in Table 1 differs from Formulation No.17 in Table 7 in the content of BHT; the content of BHT in Formulation No.17 is 4 to 5 times that in Formulation No.2. However, an increase in BHT content did not reduce the content of impurity SJ003-139; instead, it showed an increasing trend, indicating that a higher content of antioxidant does not necessarily lead to better results.

### Comparative Example 1: Investigation of the impurity content in marketed Brintellix tablets with the addition of BHT and sodium carbonate.

**Table 8 Vortioxetine Hydrobromide Tablets**

| **Ingredient** | Comparative Example 1-1 (mg/tablet) | Comparative Example 1-2 (mg/tablet) | Comparative Example 1-3 (mg/tablet) | Comparative Example 1-4 (mg/tablet) |
|---|---|---|---|---|
| Vortioxetine Hydrobromide | 12.71 | 12.71 | 12.71 | 12.71 |
| Mannitol | 61.29 | 62.49 | 60.99 | 62.79 |
| Microcrystalline Cellulose | 61 | 61 | 61 | 61 |
| Hydroxypropyl Cellulose | 3 | 3 | 3 | 3 |
| Sodium Starch Glycolate | 7.5 | 7.5 | 7.5 | 7.5 |
| Magnesium Stearate | 3 | 3 | 3 | 3 |
| Anhydrous Sodium Carbonate | 1.5 | - | 1.5 | - |
| BHT | - | 0.3 | 0.3 | - |
| Total | **150** | **150** | **150** | **150** |

Table 8 shows that Comparative Examples 1-1, 1-2, and 1-3 are tablet formulations prepared by adding anhydrous sodium carbonate and/or BHT to marketed Brintellix tablets, and are compared with Comparative Example 1-4, which is the original manufacturer formulation. The impurity contents of the initial tablets were tested: the content of impurity SJ003-139 in Comparative Example 1-1 was 1.22 ppm; that in Comparative Example 1-2 was 1.18 ppm; that in Comparative Example 1-3 was 1.15 ppm; and that in Comparative Example 1-4 was 4.0 ppm. By preparing the original manufacturer formulation without alkaline excipients and/or antioxidants, the content of impurity SJ003-139 was higher than that in formulations containing alkaline excipients and/or antioxidants, indicating that alkaline excipients and/or antioxidants can effectively reduce the content of nitrosamine impurities in tablets.

### Comparative Example 2

**Table 9 Formulations of Tablets Prepared from Vortioxetine Hydrobromide and Excipients Containing Nitrite Ions**

| **Ingredient** | Comparative Example 2-1 (mg/tablet) | Comparative Example 2-2 (mg/tablet) | Comparative Example 2-3 (mg/tablet) | Comparative Example 2-4 (mg/tablet) |
|---|---|---|---|---|
| Vortioxetine Hydrobromide | 25.42 | 25.42 | 25.42 | 25.42 |
| Microcrystalline Cellulose PH102 | 70.96 | 72.384 | 70.60 | 72.74 |
| Hydroxypropyl Cellulose | 7.12 | 7.12 | 7.12 | 7.12 |
| Anhydrous Lactose | 72.72 | 72.72 | 72.72 | 72.72 |
| Anhydrous Sodium Carbonate | 1.78 | - | 1.78 | - |
| BHT | - | 0.356 | 0.356 | - |
| **Total** | **178** | **178** | **178** | **178** |

In the above table, the content of impurity SJ003-139 is 1.25 ppm in Comparative Example 2-1, 1.20 ppm in Comparative Example 2-2, 1.16 ppm in Comparative Example 2-3, and 4.5 ppm in Comparative Example 2-4. Tablets were prepared by replacing vortioxetine hydrobromide with vortioxetine hydrochloride in Comparative Example 2-4, and the content of impurity SJ003-139 was determined to be 6.74 ppm.

The anhydrous lactose, microcrystalline cellulose, and hydroxypropyl cellulose in Table 9 are common excipients containing nitrite ions as reported in the literature: Wu, et al., Reactive Impurities in Excipients: Profiling, Identification and Mitigation of Drug-Excipient Incompatibility, AAPS PharmSciTech, 2011, 12(4), 1248-1263. The marketed Brintellix tablets also contain microcrystalline cellulose and hydroxypropyl cellulose, and these two excipients tend to form nitrosamine impurities. The test results for the content of impurity SJ003-139 in the four tablets in Table 9 demonstrate that both anhydrous sodium carbonate and BHT can inhibit the reaction between nitrite ions in these three excipients and vortioxetine to form nitrosamine impurities.

### Equivalents and Scope

Some non-limiting preferred embodiments of the present disclosure have been described above. A person skilled in the art may make various changes and modifications to this description without departing from the essential scope of the invention as defined by the claims. Such changes and modifications are also deemed to fall within the protection scope of the present invention.

## Claims

1. A pharmaceutical composition, comprising vortioxetine or a pharmaceutically acceptable salt thereof and alkaline excipient, wherein the content of nitrosamine impurity in the pharmaceutical composition is not more than 20 ppm, and the structural formula of the nitrosamine impurity is as shown in Formula SJ003-139:

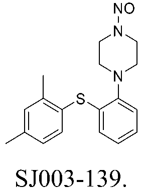

2. The pharmaceutical composition according to claim 1, wherein the alkaline excipient is at least one selected from trisodium phosphate, disodium hydrogen phosphate, tripotassium phosphate, dipotassium hydrogen phosphate, calcium phosphate, calcium carbonate, potassium carbonate, sodium carbonate, sodium bicarbonate, potassium citrate, sodium acetate, calcium hydroxide, sodium hydroxide, potassium hydroxide or meglumine, preferably sodium carbonate.

3. The pharmaceutical composition according to claim 1 or 2, further comprising an antioxidant with phenol structure, butylated hydroxytoluene or butylated hydroxyanisole, preferably butylated hydroxytoluene.

4. The pharmaceutical composition according to any one of claims 1 to 3, wherein the content of nitrosamine impurity in the pharmaceutical composition is not more than 19 ppm, not more than 18 ppm, not more than 17 ppm, not more than 16 ppm, not more than 15 ppm, not more than 14 ppm, not more than 13 ppm, not more than 12 ppm, not more than 11 ppm, not more than 10 ppm, not more than 9 ppm, not more than 8 ppm, not more than 7 ppm, not more than 6.7 ppm, not more than 6 ppm, not more than 5 ppm, not more than 4 ppm, not more than 3 ppm, or not more than 2 ppm.

5. The pharmaceutical composition according to claim 4, wherein the content of nitrosamine impurity in the pharmaceutical composition is not more than 6.7 ppm, not more than 6 ppm, not more than 5 ppm, not more than 4 ppm, not more than 3 ppm, or not more than 2 ppm.

6. The pharmaceutical composition according to any one of claims 1 to 5, further comprising two or more of the following pharmaceutically acceptable excipients: a filler, a disintegrant, a glidant, a lubricant, wherein
the filler is selected from one or two of mannitol, sorbitol, xylitol, polyethylene glycol, hydroxypropyl cellulose, hypromellose, microcrystalline cellulose, anhydrous lactose, preferably one or two of mannitol or microcrystalline cellulose, more preferably mannitol;
the disintegrant is selected from at least two of crospovidone, croscarmellose sodium, sodium carboxymethyl starch, preferably crospovidone and croscarmellose sodium;
the glidant is selected from at least two of talc, silicon dioxide, magnesium silicate, preferably talc and silicon dioxide;
the lubricant is selected from at least one of magnesium stearate, stearic acid, sodium lauryl sulfate, talc, preferably magnesium stearate;
optionally, the pharmaceutical composition further comprises at least one of a flavoring agent and a taste masking agent, wherein the flavoring agent is selected from at least one of L-menthol, neotame, monosodium glutamate, saccharin, preferably L-menthol or neotame; the taste masking agent is selected from at least one of amino methacrylate copolymer, polyacrylic resin II, polyacrylic resin III, polyvinyl acetate phthalate, preferably amino methacrylate copolymer.

7. The pharmaceutical composition according to any one of claims 1 to 6, wherein the pharmaceutical composition comprises:
vortioxetine or a pharmaceutically acceptable salt thereof 10~20%;
alkaline excipient 0.2~5%;
antioxidant 0.02~0.1%;
filler 30~85%;
disintegrant 1.5~15%;
glidant 1.0~12%;
lubricant 0.25~5%;
taste masking agent 1~25%.

8. The pharmaceutical composition according to any one of claims 1 to 6, wherein the pharmaceutical composition comprises:
vortioxetine or a pharmaceutically acceptable salt thereof 10~20%;
anhydrous sodium carbonate 0.2~5%;
butylated hydroxytoluene 0.02~0.1%;
mannitol 30~85%;
crospovidone 1~5%;
croscarmellose sodium 0.5~8%;
talc 1~10%;
Silicon dioxide 0.1~1.0%;
magnesium stearate 0.25~5%;
amino methacrylate copolymer 1~25%;
wherein the pharmaceutically acceptable salt is hydrochloride, hydrobromide or hemihydrobromide.

9. The pharmaceutical composition according to any one of claims 1 to 8, wherein the pharmaceutical composition is oral solid preparation, preferably is tablet or granule, more preferably is orally disintegrating tablet.

10. A method for treating depression disorder or cognitive impairment in a subject, comprising administering to the subject the pharmaceutical composition according to claim 1~9.
